# EUROPEAN PATENT APPLICATION

(11) **EP 2 105 153 A2**
(43) Date of publication of application: **30.09.2009**
(21) Application number: 09250872.0
(22) Date of filing: 27.03.2009
(51) Int. Cl.: A61M 5/142

(54) **Medical device mechanical pump**

(30) Priority: 27.03.2008 US 40024 P
(71) Applicant: LifeScan, Inc., Milpitas, CA 95035 (US)
(72) Inventor: Krulevitch, Peter, Pleasanton, CA 94566 (US); Wilk, Robert, Sierra Village, CA 95346 (US); O'Connor, Sean M., West Chester, PA 19380 (US); Zhao, Mingqi, San Jose, CA 95129 (US); Sieh, Zara, Pleasanton, CA 94566 (US); Savage, Donna, Rolling Hills Estates, CA 90274 (US)
(74) Representative: Tunstall, Christopher Stephen

(57) **Abstract**

A medical device pump (100) with a housing with a compartment (150) for removably receiving a cartridge containing a therapeutic agent, a conduit (160) configured to operatively provide a fluid flow path for therapeutic agent to exit from the cartridge, a user activated delivery button (110), a trigger mechanism (310), and a mechanical pump mechanism. The trigger mechanism, user activated delivery button and mechanical pump mechanism of the medical device pump are configured such that the trigger mechanism is activated by a user fully activating the user activated delivery button. Moreover, such full activation generates mechanical power employed by, and sufficient for, the mechanical pump mechanism to pump a predetermined volume of therapeutic agent from the cartridge and through the fluid flow path.

## Description

### FIELD OF THE INVENTION

The present invention relates to medical devices, and, more particularly, to medical device mechanical pumps for delivering therapeutic agents. Embodiments of the present device are useful for medical drug delivery devices, including small, low cost insulin delivery devices worn on the skin for treating Type 1 and Type 2 diabetes.

### BACKGROUND OF THE INVENTION

Transcutaneous delivery of medicine is an alternative to orally delivered pharmaceuticals, which reach the blood stream by way of the intestines. Some medicines lose their effectiveness when ingested and must be delivered using other means. Parenteral delivery refers to delivery of medicine to the body by means other than via the intestines. Intradermal, subcutaneous, and intravenous injections are examples of parenteral delivery.

Insulin is an example of a medication that must be administered using parenteral delivery. Insulin is injected by patients with Type 1 diabetes, and some patients with Type 2 diabetes. In Type 1, or juvenile onset diabetes, the pancreas no longer produces insulin, and insulin must be injected to regulate blood sugar. In Type 2 diabetes, the body loses its sensitivity to insulin, and more insulin is required to regulate blood sugar. In the later stages of the disease, the pancreas of a Type 2 diabetic stops producing insulin, and the patient becomes insulin dependent, similar to a Type 1 diabetic.

Different patients adopt different insulin regimens, depending on many factors, including the type and stage of the disease, access to medical care, support by family members, lifestyle, motivation, and attitude toward the disease. A healthy pancreas secretes insulin at a low, steady basal or background rate, and produces larger boluses of insulin in response to food intake. Injections of long acting insulin (formulated to be taken up by the body slowly and steadily over a period of several hours) are used to mimic basal insulin, and injections of rapid acting insulin (formulated to be taken up quickly) are used for boluses. Type 1 diabetics and insulin dependent Type 2 diabetics typically adopt regimens that include both basal and bolus insulin. Type 2 diabetics new to insulin might start on a relatively low dosage of basal insulin only, with one shot a day of long acting insulin. Alternatively, new to insulin Type 2 diabetics might start on a relatively low dosage of bolus only insulin, taken one to two times a day with meals. With time, Type 2 diabetics will increase their insulin dosage and add bolus or basal insulin to complement their initial insulin regimen.

Typically, the insulin is drawn up from a vial and injected with a syringe and needle. Insulin therapy with syringes and vials is low in cost but requires significant skill and dexterity to draw up the proper amount of insulin and purge bubbles. Insulin pens are popular in Europe and are beginning to displace syringes in the United States. Insulin pens are comprised of a prefilled insulin cartridge with a plunger, a needle, a mechanism that allows the user to dial in the specific amount of insulin to be delivered, and a button to inject the insulin. Such pens are, in essence, hand-held medical fluid delivery devices. Disposable insulin pens with an integrated insulin cartridge and reusable insulin pens with replaceable insulin cartridges are both available on the market today. Insulin pens are convenient and easy to use, eliminating the need to draw up the insulin into a syringe, allowing the patient to set the dose by turning a dial rather than trying to read a meniscus in a small, finely graduated syringe, and simplifying the elimination of bubbles, which affect delivery accuracy.

Continuous subcutaneous insulin infusion (CSII), or insulin pump therapy, is a preferred method for delivering insulin to diabetic patients, and is known to have certain advantages over injection of insulin with syringes or pens. Insulin pump therapy consists of a low, steady basal delivery, with larger bolus deliveries taken in conjunction with food intake, a pattern that closely resembles insulin secretion from a healthy pancreas. Instead of using long acting insulin for basal delivery, insulin pumps deliver small shots of rapid acting insulin at regular time intervals to approximate slow continuous delivery. Recently introduced "smart pumps" have features that keep track of insulin injection history, remember commonly used dosages, help calculate bolus size, and allow for fine-tuning of basal and bolus delivery. Insulin pumps also offer increased lifestyle flexibility through frequent, convenient insulin dosing, allowing the user to eat what they want when they want and still maintain control of their blood glucose levels.

Insulin pumps are comprised of the pump engine, an insulin reservoir, and an infusion set which delivers insulin from the pump across the skin to the patient. The infusion set may be worn for multiple days, allowing infusion of insulin across the skin as required without the need to pierce the skin multiple times with needles for individual injections. The pump may be worn on a belt clip, or placed in a pants pocket, holster, or bra, for example. The pump is connected to the user via an infusion set, comprised of a transcutaneously inserted cannula affixed to the body with an adhesive patch, with a length of plastic tubing linking the cannula to the pump. The cannula is usually attached to the user's abdomen region, although other locations such as the lower back or thigh may be used. A new generation of pumps known as patch pumps is now beginning to appear on the market. These pumps are smaller in size and affix directly to the skin such that the tubing leading to the cannula is shortened or eliminated entirely.

There are several problems associated with existing approaches to insulin delivery, and insulin therapy in general. Even though insulin therapy is known to be the best way to limit glycemic excursions, Type 2 patients resist starting insulin. Many patients associate insulin with the last stages of the disease leading to death, they are afraid of needles and giving themselves injections, and the therapy is complicated and confusing, involving carbohydrate counting, regulation of food intake, and the relationship between insulin, food, and exercise. Physicians delay putting their Type 2 patients on insulin because the patients are resistant, it is difficult and time-consuming to initiate and manage patients on insulin therapy, and they are afraid of dangerous and potentially fatal hypoglycemic events induced by delivering too much insulin. This delay in starting insulin therapy accelerates the course of the disease.

As mentioned above, the needles used with syringe and pen injections are intimidating to patients. Some patients have needle-phobia and just the thought of injecting causes anxiety. For both syringes and pens, the patient must remember to carry the insulin and supplies with them if they are going to inject away from home. Syringes require the user to draw up the insulin from a vial and purge bubbles, a multi-step process requiring significant skill, dexterity, and visual acuity to perform accurately. In addition, syringes offer no means for creating a time record of delivery, other than relying on the patient to keep a logbook.

Insulin pens solve many of the problems associated with syringes, greatly simplifying the injection process. However, they still require the use of needles, and can be inaccurate if the patient does not prime the pen before injecting or does not keep the needle in the skin and hold the button down for a sufficient length of time during the injection. Recently introduced smart pens keep a primitive record of the most recent injections, but cannot distinguish priming shots from regular injections, and do not allow for downloading and analysis of the insulin data in conjunction with blood glucose data.

While insulin pumps offer many benefits relative to syringes and pens, they also have several problems. Insulin pumps are expensive, complex devices with many features, requiring multiple steps to set up and use. Thus they are difficult for health care providers to learn and teach, and for patients to learn and use. Conventional insulin pumps use indirect pumping in which a motor and gears drive a lead screw, which pushes on a plunger in a syringe-like cartridge to inject the insulin. The indirect pumping approach is susceptible to over-delivery of insulin due to siphoning and pressure differentials.

Bubbles present a major challenge with conventional insulin pumps, which rely on the user to fill a syringe-like reservoir. It is difficult for the user to purge all of the air out of the system when setting up the pump, and additional bubbles can form when dissolved gas in the insulin comes out of solution due to changes in temperature or pressure. During delivery, bubbles displace insulin and reduce delivery accuracy. For example, a small 10 microliter bubble passing through the pump to the user is equivalent to 1 unit of missed insulin. Pre-filled insulin cartridges come to the user with approximately 20 - 40 microliters of air in the cartridge, and more gas can come out of solution during use.

Endocrinologists prescribe most insulin pumps, but many diabetics only see primary care physicians (PCPs). Many physicians, including endocrinologists and PCPs, are unwilling to put their patients on pumps because they don't think their patients can handle it, or because it will cause more work for the physicians that they are not reimbursed for. The pumps are relatively large, making them difficult to wear and operate discretely. Insulin pump therapy is expensive, with conventional pumps costing approximately $5,000 up front. A low cost pump could make insulin pump therapy more accessible, but it is important to provide the accuracy and critical safety benefits of conventional pumps such as occlusion detection and delivery confirmation. Furthermore, it would be beneficial to maintain a delivery record for retrospective analysis by the physician and/or patient.

For these reasons, currently available insulin pumps are used predominantly by a small class of insulin-using diabetics - sophisticated Type 1 patients who meticulously monitor their blood glucose levels and are proficient at counting carbohydrates to determine insulin dosing. Many people who could benefit from insulin pump therapy, such as Type 2 diabetics, are unable to use them or choose not to use them because of the disadvantages discussed above.

Thus, it is desirable to have an insulin pump that is low in cost, accurate, safe, and simple enough to teach and use that primary care physicians (PCPs) would put their insulin using Type 1 and Type 2 patients on the pump, allowing more diabetics to benefit from the advantages of insulin pump therapy. The device would have the simplicity and low cost of an insulin pen, combined with the convenience, lifestyle benefits, data logging, and safety features of a pump.

### SUMMARY

The following is a non-exhaustive list of embodiments of the invention that are or may be claimed.
1. A medical device pump comprising:
   a housing with a compartment therein, the compartment configured for accommodating a cartridge containing a therapeutic agent;
   a conduit configured to operatively provide a fluid flow path for therapeutic agent to exit from the cartridge;
   a user activated delivery button;
   a trigger mechanism, and
   a mechanical pump mechanism,
   wherein the trigger mechanism, user activated delivery button and mechanical pump mechanism are configured such that the trigger mechanism is activated by a user fully activating the user activated delivery button and such full activation generates mechanical power employed by, and sufficient for, the mechanical pump mechanism to pump a predetermined volume of therapeutic agent from the cartridge into the fluid flow path.
2. The medical device pump of embodiment 1 wherein the trigger mechanism is configured such that partial activation of the user activated delivery button by a user is insufficient to activate the trigger mechanism to generate mechanical power for the mechanical pump mechanism and, therefore, results in zero volume of therapeutic agent being pumped by the mechanical pump mechanism.
3. The medical device pump of embodiment 1 wherein the trigger mechanism is configured such that once a user has fully activated the user activated delivery button and the trigger mechanism has been activated, the user activated delivery button must be fully released before the trigger mechanism can be again activated.
4. The medical device pump of embodiment 1 further including:
   a user activated safety release button configured such that pressing of the safety release button by a user enables the user activated delivery button to be fully activated by a user.
5. The medical device pump of embodiment 1 wherein the housing is configured for attachment to an adhesive patch platform.
6. The medical device pump of embodiment 1 further including a bubble trap disposed in the fluid flow path, the bubble trap configured to prevent inadvertent bubbles in the cartridge from reaching the mechanical pump mechanism.
7. The medical device pump of embodiment 1 further including:
   a one-way communication means, the one-way communication means configured to operatively send a communication signal from the medical device pump to an associated meter configured to detect the communication signal.
8. The medical device pump of embodiment 7 wherein the one-way communication means includes at least one Radio Frequency Identification (RFID) tag.
9. The medical device pump of embodiment 7 wherein full activation of the user activated delivery button interrupts the sending of a communication signal from the one-way communication means to the associated meter.
10. The medical device pump of embodiment 7 wherein full activation of the user activated delivery button initiates the sending of a communication signal from the one-way communication means to the associated meter.
11. The medical device pump of embodiment 7 wherein the one-way communication means is configured to be externally powered by the associated meter.
12. The medical device pump of embodiment 7 wherein the one-way communication means includes at least two RFID tags, and
   wherein full activation of the user activated delivery button interrupts the sending of a communication signal from one of the at least two RFID tags to the associated meter, and
   wherein full activation of the user activated delivery button also initiates the sending of a communication signal from another of the at least two RFID tags to the associated meter.
13. The medical device pump of embodiment 7 wherein the communication signal enables the associated meter to create a time record of therapeutic agent pumped by the mechanical pump mechanism.
14. The medical device pump of embodiment 1 wherein the compartment of the housing is configured to accommodate a cartridge in the form of a user-filled reservoir.
15. The medical device pump of embodiment 1 wherein the compartment of the housing compartment is configured to removably accommodate the cartridge.
16. The medical device pump of embodiment 1 wherein the compartment of the housing is configured to accommodate a disposable pre-filled cartridge.
17. The medical device pump of embodiment 1 wherein the medical device is configured for multiple full activations of the user activated delivery button.
18. The medical device pump of embodiment 1 wherein the conduit and mechanical pump mechanism have a predetermined compliance that is sufficiently low that the user activated delivery button can not be fully activated by a user in the event of an operative occlusion in at least one of the conduit and mechanical pump mechanism.
19. A method for transcutaneously delivering a therapeutic agent to a patient comprising:
   inserting a cartridge pre-filled with a therapeutic agent into a cartridge compartment of a medical device pump;
   priming the medical device pump; and
   transutaneously delivering the therapeutic agent to a patient by action of the patient fully activating a user activated delivery button of the medical device pump, thus activating a trigger mechanism of the medical device pump and generating mechanical power employed by, and sufficient for, a mechanical pump mechanism of the medical device pump to pump a predetermined volume of therapeutic agent from the cartridge and into a fluid flow path of the medical device pump.
20. The method of embodiment 19 wherein the therapeutic agent is insulin.
21. The method of embodiment 19 further including the step of:
   attaching the medical device pump to a disposable infusion set prior to the delivering step.
22. The method of embodiment 20 wherein the attaching step includes attaching the medical device pump to an adhesive patch platform of a disposable infusion set.
23. The method of embodiment 19 further including, prior to the delivering step, the step of:
   pressing, by the patient, of a user activated safety release button of the medical device pump, the user activated safety release button configured such that pressing of the safety release button by a user enables the user activated delivery button to be activated by a user.
24. The method of embodiment 19 further including, following the delivering step, the step of:
   releasing fully the user activated delivery button by the patient; and subsequently
   transutaneously delivering a further predetermined volume of therapeutic agent to the patient by action of the patient again fully activating the user activated delivery button of the medical device pump, thus activating the trigger mechanism of the medical device pump and generating mechanical power employed by, and sufficient for, the mechanical pump mechanism of the medical device pump to again pump the predetermined volume of therapeutic agent from the cartridge and into the fluid flow path of the medical device pump.
25. The method of embodiment 19 further including the step of:
   sending a communication signal from the medical device pump to an associated meter using a one-way communication means of the medical device pump.
26. The method of embodiment 25 wherein during the sending step, full activation of the user activated delivery button interrupts the sending of the communication signal from the one-way communication means to the associated meter.
27. The method of embodiment 25 wherein the sending step is initiated by the patient's full activation of the user activated delivery button.
28. The method of embodiment 25 wherein during the sending step, the one-way communication means is externally powered by the associated meter.
29. The method of embodiment 19 wherein the fluid flow path includes a bubble trap and the transcutaneously delivering step includes subcutaneously delivering the therapeutic agent.
30. The method of embodiment 19 wherein the fluid flow path includes a bubble trap and the transcutaneously delivering step includes intradermally delivering the therapeutic agent.
31. A medical device kit comprising:
   a medical device pump that includes;
      a housing with a compartment therein, the compartment configured for removably receiving a cartridge containing a therapeutic agent;
      a conduit configured to operatively provide a fluid flow path for therapeutic agent to exit from the cartridge;
      a user activated delivery button;
      a trigger mechanism, and
      a mechanical pump mechanism,
   wherein the trigger mechanism, user activated delivery button and mechanical pump mechanism are configured such that the trigger mechanism is activated by a user fully activating the user activated delivery button and such full activation generates mechanical power employed by, and sufficient for, the mechanical pump mechanism to pump a fixed volume of therapeutic agent from the cartridge into the fluid flow path; and a meter configured to detect a communication signal from the medical device pump.
32. The medical device kit of embodiment 31 further including an adhesive patch platform and wherein the housing is configured for removable attachment to the adhesive patch platform.
33. The medical device kit of embodiment 32 wherein the adhesive patch platform includes a flexible cannula inserter and a flexible cannula.
34. The medical device kit of embodiment 33 wherein the trigger mechanism, user activated delivery button and mechanical pump mechanism are configured such that the trigger mechanism is activated by a user fully activating the user activated delivery button and such full activation generates mechanical power employed by, and sufficient for, the mechanical pump mechanism to pump a predetermined volume of therapeutic agent from the cartridge, through the fluid flow path into the flexible cannula.
35. The medical device kit of embodiment 31 wherein the therapeutic agent is insulin.
36. The medical device kit of embodiment 31 further including a cartridge containing a therapeutic agent.
37. The medical device kit of embodiment 36 wherein the cartridge is configured to be pre-filled prior to being provided to the user.
38. The medical device kit of embodiment 36 wherein the cartridge is configured to be filled by a user of the kit.
39. The medical device kit of embodiment 31 wherein the trigger mechanism is configured such that partial activation of the user activated delivery button by a user is insufficient to activate the trigger mechanism to generate mechanical power for the mechanical pump mechanism and, therefore, results in zero volume of therapeutic agent being pumped by the mechanical pump mechanism.
40. The medical device kit of embodiment 31 wherein the trigger mechanism is configured such that once a user has fully activated the user activated delivery button and the trigger mechanism has been activated, the user activated delivery button must be fully released before the trigger mechanism can be again activated.
41. The medical device kit of embodiment 31 further including:
   a user activated safety release button configured such that pressing of the safety release button by a user provides for the user activated delivery button to be activated by a user.
42. The medical device kit of embodiment 31 wherein the housing is configured for attachment to an adhesive patch platform.
43. The medical device kit of embodiment 31 further including a bubble trap disposed in the fluid flow pathway, the bubble trap configured to prevent inadvertent bubbles in the cartridge from reaching the mechanical pump mechanism.
44. The medical device kit of embodiment 31 further including:
   a one-way communication means, the one-way communication means configured to operatively send a communication signal from the medical device pump to the associated meter.
45. The medical device kit of embodiment 44 wherein the one-way communication means includes at least one Radio Frequency Identification (RFID) tag.
46. The medical device kit of embodiment 44 wherein full activation of the user activated delivery button interrupts the sending of a communication signal from the one-way communication means to the associated meter.
47. The medical device kit of embodiment 44 wherein full activation of the user activated delivery button initiates the sending of a communication signal from the one-way communication means to the associated meter.
48. The medical device kit of embodiment 44 wherein the one-way communication means is configured to be externally powered by the associated meter.
49. The medical device kit of embodiment 44 wherein the one-way communication means includes at least two RFID tags, and
   wherein full activation of the user activated delivery button interrupts the sending of a communication signal from one of the at least two RFID tags to the associated meter, and
   wherein full activation of the user activated delivery button initiates the sending of a communication signal from another of the at least two RFID tags to the associated meter.
50. A hand-held medical fluid delivery device comprising:
   a housing with a compartment therein, the compartment configured for removably receiving a cartridge containing a therapeutic agent;
   a conduit configured to operatively provide a fluid flow path for therapeutic agent to exit from the cartridge;
   a user activated delivery button,
   a mechanical pump mechanism, and
   a one-way communication means, the one-way communication means configured to operatively send a communication signal from the hand-held medical fluid delivery device to an associated meter configured to detect the communication signal,
   wherein the user activated delivery button and mechanical pump mechanism are configured such that a user fully activating the user activated delivery button generates mechanical power employed by, and sufficient for, the mechanical pump mechanism to pump a predetermined volume of therapeutic agent from the cartridge into the fluid flow path.
51. The hand-held medical fluid delivery device of embodiment 50 wherein the conduit and fluid path are configured to subcutaneously deliver therapeutic agent to the user.
52. The hand-held medical fluid delivery device of embodiment 50 wherein the conduit and fluid path are configured to intradermally deliver therapeutic agent to the user.
53. The hand-held medical fluid delivery device of embodiment 50 further including a bubble trap disposed in the fluid flow path, the bubble trap configured to prevent inadvertent bubbles in the cartridge from reaching the mechanical pump mechanism.
54. The hand-held medical fluid delivery device of embodiment 50 further including a priming sensor configured to detect priming and distinguish priming from subsequent fluid delivery.
55. A medical fluid delivery device comprising:
   a housing with a compartment therein, the compartment configured for accommodating a therapeutic agent reservoir;
   a conduit configured to operatively provide a fluid flow path for therapeutic agent to exit from the reservoir;
   a mechanical pump mechanism; and
   a bubble trap disposed in the fluid flow path, the bubble trap configured to prevent inadvertent bubbles in the cartridge from reaching the mechanical pump mechanism.
56. The medical fluid delivery device of embodiment 55
   wherein the conduit and fluid path are configured to subcutaneously deliver therapeutic agent to the user.
57. The medical fluid delivery device of embodiment 55
wherein the conduit and fluid path are configured to intradermally deliver therapeutic agent to the user.

### BRIEF DESCRIPTION OF THE DRAWINGS

Figure 1 is a simplified perspective view depiction of a medical device mechanical pump according to an embodiment of the present invention, an infusion set patch, and a pre-filled cartridge;
Figure 2 is a simplified perspective view depiction of the medical device mechanical pump of Figure 1 with the pre-filled cartridge inserted and attached to the infusion set patch;
Figure 3 is a simplified perspective view depiction the medical device mechanical pump of Figure 1 with the upper housing removed, revealing the inner components of the medical device mechanical pump;
Figure 4 is a simplified cross section of a mechanical pump engine (also referred to herein as a "micropump") with integrated bubble trap that draws fluid from the pre-filled cartridge and can be employed in various embodiments of the present invention.
Figure 5 is a simplified exploded perspective view of a micropump actuation triggering mechanism as can be employed in various embodiments of the present invention;
Figure 6 is a simplified exploded perspective view of a micropump actuation triggering mechanism as can be employed in various embodiments of the present invention;
Figure 7 is a simplified exploded perspective view of the micropump actuation triggering mechanism of Figure 6 from another viewpoint; and
Figure 8 is a simplified depiction of a passive circuit to enable one-way communication from a medical device insulin pump according to an embodiment of the present invention and an associated blood glucose meter.

### DETAILED DESCRIPTION

The present invention relates to medical device mechanical pumps, and, more particularly, to medical device mechanical pumps (also referred to herein as a "mechanical pump" and/or a "medical device pump") for delivering therapeutic agents. Although a simple mechanical patch pump for delivering insulin or other therapeutic agents is described for the purpose of example, one of skill in the art would understand that other embodiments of this device could be used for other devices that would benefit from a mechanical pump, such as hand-held insulin pens (a type of portable user-operated medical fluid delivery device), more complex insulin pumps with additional features, belt or pocket worn insulin pumps, and medical fluid delivery devices for delivering other therapeutic agents such as drugs or other fluids for other applications such as for treating pain.

One aspect of the present invention is to provide an easy to teach, easy to learn, easy to use mechanical pump for delivering insulin. The simple mechanical pump does not require electronics or battery power for delivering insulin, instead relying on power provided by the user when pressing the delivery button. Another aspect of the present invention is to provide a pump that delivers discrete shots of a fixed size with each button press that can be used to deliver long acting basal insulin, regular or rapid acting insulin for boluses, or a mix of long acting and regular or rapid acting insulin for basal/bolus therapy. Another aspect of the present invention is to provide a low cost insulin patch pump comprised of a disposable patch pump that accepts pre-filled cartridges and attaches to a disposable infusion set. Another aspect of the present invention is to provide a low cost mechanical pump that provides beneficial safety features and accuracy. Another aspect of the present invention is to provide a simple, low cost means of communication from the disposable pump to a blood glucose meter to confirm and record insulin delivery events.

The mechanical pump disclosed herein is useful for delivering insulin to diabetic patients, and also may be used for delivering other drugs, cells, genetic material such as DNA, and biopharmaceuticals including protein-based drugs, for applications such as treatment for diabetes, Parkinson's disease, epilepsy, pain, immune system diseases, inflammatory diseases, and obesity (referred to generally as therapeutic agents).

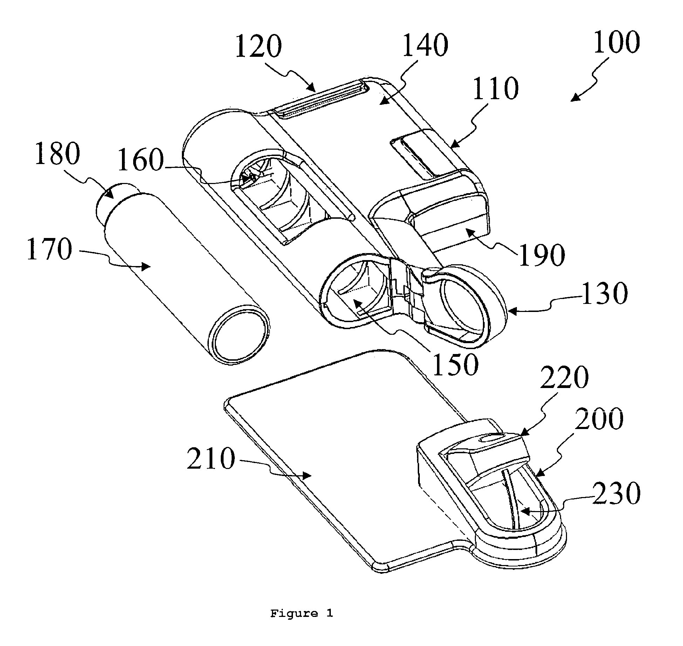

The mechanical pump, generally denoted by 100 in Figure 1, accepts pre-filled insulin cartridge 170 and docks onto adhesive patch platform 210. Using a pre-filled insulin cartridge greatly simplifies the pump set up for the patient, eliminating the need to draw up insulin from a vial and purge bubbles. In a preferred embodiment of the present invention, mechanical pump 100 is affixed directly to the skin via adhesive patch platform 210. Mechanical pump 100 is simple in nature and has minimal features evident from the outside. These include insulin cartridge compartment 150, cartridge door 130, lower housing 190 and upper housing 140, safety release button 120 and delivery button 110. Upon inserting insulin cartridge 170 into insulin cartridge compartment 150 and closing cartridge door 130, conduit 160 penetrates septum 180, providing access to insulin inside cartridge 170. Attached to adhesive patch platform 210 is flexible cannula inserter 200 with inserter lever 220 and flexible cannula 230.

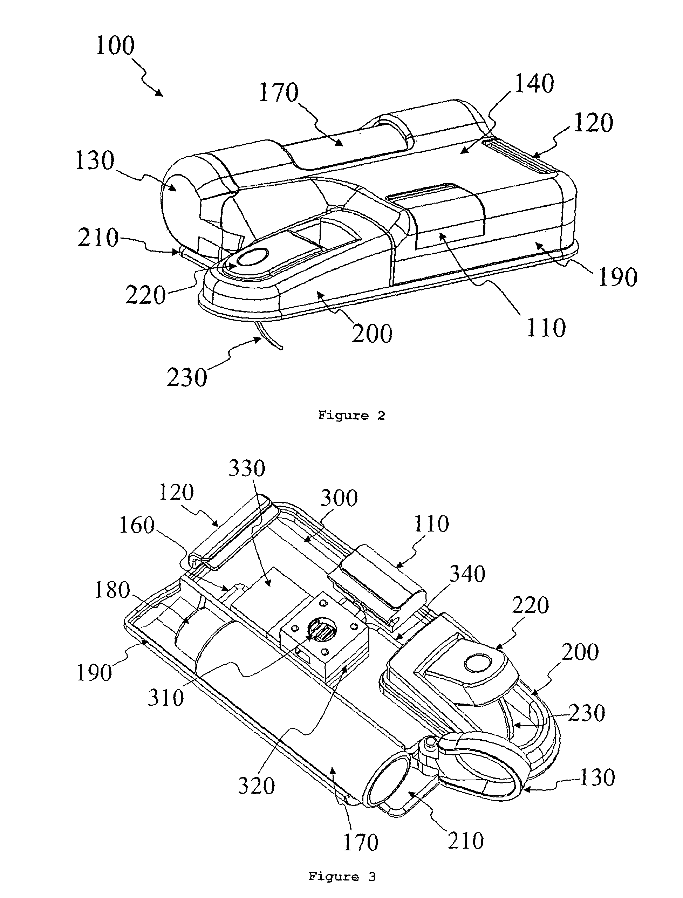

Referring now to Figure 2, mechanical pump 100 is shown attached to adhesive patch platform 210 with insulin cartridge 170 inserted into insulin cartridge compartment 150 and cartridge door 130 closed. Flexible cannula inserter lever 220 is in the down position, with flexible cannula 230 protruding from the bottom side of adhesive patch platform 210.

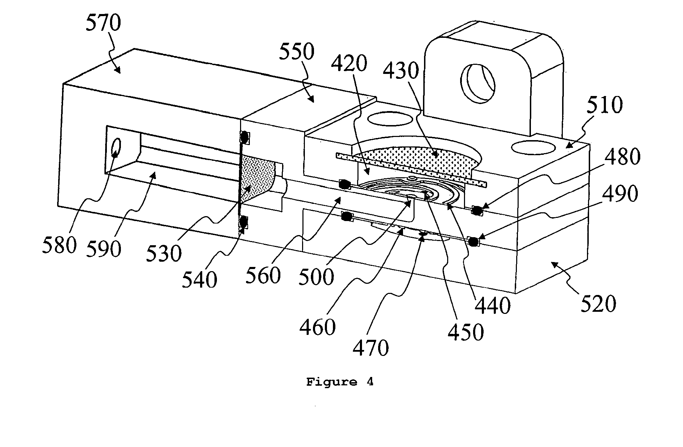

Figure 3 shows mechanical pump 100 with upper housing 140 removed to reveal internal components, which are shown in detail in Figure 4, Figure 5, and Figure 6. Pressing safety release button 20 slides safety release rod 300 forward, allowing delivery button 110 to be pressed in. Pressing delivery button 110 activates trigger mechanism 310, which in turn generates a single stroke from micropump 320. Stroke from micropump 320 first generates a pressure drop that sucks in insulin from insulin cartridge 170 via conduit 160, and through bubble trap 330, then delivers insulin via delivery conduit 340 to flexible conduit 230 and across the skin to the patient.

Focusing now on Figure 4, a cross section of micropump 320 and bubble trap 330, a description of the pump mechanism is provided. Pressing down on flexible diaphragm 430 pressurizes pump chamber 420, closing inlet valve 440, which seals pump inlet 500, opening outlet valve 460, and delivering fluid through channel 450 to pump outlet 470. The volume of fluid delivered to pump outlet 470 is equivalent to the volume of pump chamber 420 displaced by flexible diaphragm 430. Allowing flexible diaphragm 430 to return to the up position as pictured in Figure 4 causes the pressure in pump chamber 420 to drop. The drop in pressure closes outlet valve 460, sealing channel 450, and lifts inlet valve 440, opening pump inlet 500 and causing fluid to fill pump chamber 420 via pump inlet channel 560.

Before entering the pump, fluid passes through bubble trap 330, which is comprised of bubble trap housing 570, bubble trap inlet 580, bubble trap chamber 590, and porous membrane 530. In a preferred embodiment, porous membrane 530 is hydrophilic and has an average pore size between approximately 0.05 and 2 microns. Hydrophobic porous material also will work for the bubble trap. Bubble trap 330 prevents bubbles originating in the insulin reservoir from reaching micropump 320, where they could increase compliance of the system and affect delivery accuracy. Bubble trap 330 also filters particles out of the system before reaching micropump 320, where they could prevent inlet valve 440 or outlet valve 460 from sealing properly. During priming, micropump 320 pumps air out of the system and fills conduit 160, bubble trap chamber 590, pump inlet channel 560, pump chamber 420, delivery conduit 340, and flexible conduit 320 with fluid from cartridge 170. The process of filling wets porous membrane 530, and subsequent bubbles released from cartridge 170 become trapped in bubble trap chamber 590. Bubble trap 330 is designed such that the volume of bubble trap chamber 590 is greater than the volume of bubbles that might exist within cartridge 170.

Continuing with Figure 4, Pump chamber o-ring 480 seals upper pump housing 510 to valve seat plate 550, lower housing o-ring 490 seals lower housing 520 to valve seat plate 550, and bubble trap o-ring 540 seals bubble trap housing 570 to back side of valve seat plate 550. Upper pump housing 510, valve seat plate 550, lower housing 520, and bubble trap housing 570 are attached to each other using screws, adhesive, pins on one side that interfere with holes on the other, heat staking, or ultrasonic welding.

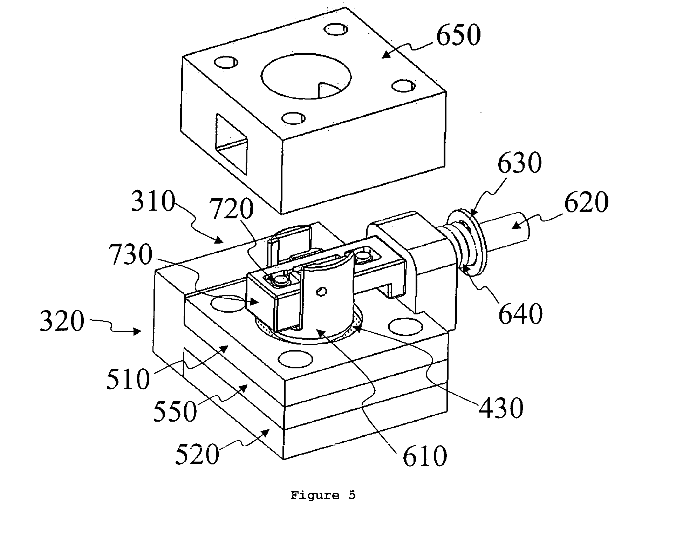

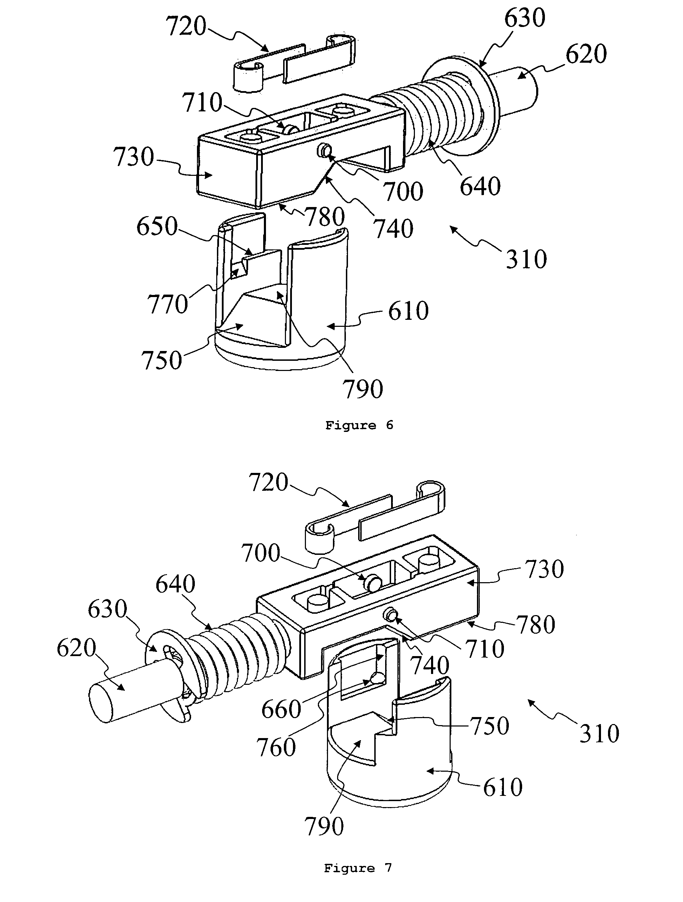

Trigger mechanism 310, shown in Figure 5 and Figure 6, serves to translate presses of delivery button 110 into pumping cycles of micropump 320. A function of trigger mechanism 310 is to ensure that partial presses of delivery button 110 cannot produce a fraction of a full pump stroke. When delivery button 110 is pushed past a specific distance, trigger mechanism 310 activates a complete pump stroke. When delivery button 110 is pushed less than the specific distance, no pump stroke is produced. Similarly, the triggering mechanism ensures that the patient must fully release delivery button 110 before pressing again to deliver another pump stroke, a feature that likewise prevents activation of a partial pump stroke.

In Figure 5, trigger mechanism 310 is shown in relation to micropump 320. Trigger mechanism housing 650 is shown removed to reveal trigger mechanism 310. Piston 610 engages flexible diaphragm 430. Activation rod 620 is attached to delivery button 110 and is biased in the out position by coil spring 640 which pushes against snap ring 630. Figure 6 and Figure 7 show components of trigger mechanism 310 in more detail from two viewing angles. Piston 610 and leaf springs 720 are shown separated from the trigger mechanism assembly for clarity. In the rest position (delivery button 110 not pressed), trigger mechanism body flat 780 contacts piston flat 790, maintaining piston in the down position such that micropump diaphragm 430 is biased downwards. Pressing delivery button 110 pushes activation rod 620, compressing coil spring 640 and pushing trigger mechanism body 730 forward. First pin 710 protrudes outwardly from trigger mechanism body 730 and rides along piston ledge 650, preventing piston from rising. First pin 710 is biased inwards by one of leaf springs 720. When first pin 710 is pushed beyond piston ledge 650, piston 610 rises and first pin 710 slides into ramped piston slot 770. When delivery button 110 is released, coil spring 640 pushes activation rod 620 back towards the rest position, and ramp 740 on trigger mechanism body 730 engages piston ramp 750, pushing piston back down to the rest position, where it is held in place with trigger mechanism body flat 780 again contacting piston flat 790. If the patient tries to re-press delivery button 110 before trigger mechanism has returned to the rest position, second pin 700, biased inwards by one of leaf springs 720, engages detent 760, stopping motion of trigger mechanism body 730 and preventing delivery of a partial bolus. Under normal operating conditions when pressing delivery button 110, second pin 700 slides over vertical piston ramp 660, allowing forward motion of trigger mechanism body 730.

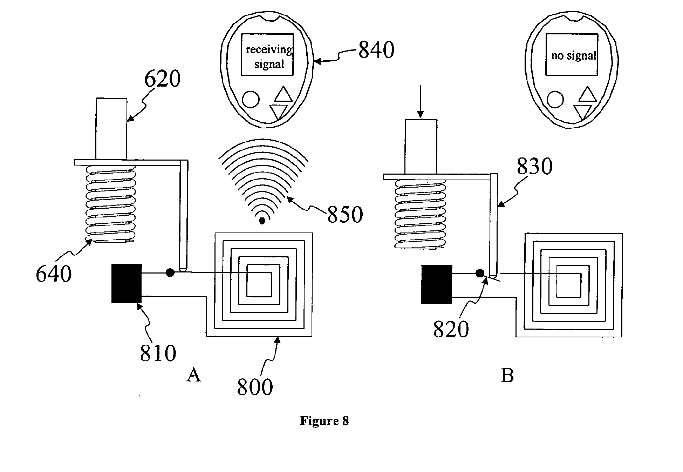

Mechanical pump 100 includes a simple, low cost, batteryless means for one-way communication to a blood glucose meter. Communication from the pump to the meter is useful for recording and time stamping insulin delivery events for later review by the patient and/or health care provider. This information can be useful to the patient, for example, to remember whether or not they have already delivered their insulin. One embodiment for communicating from mechanical pump 100 to a blood glucose meter is shown in Figure 8 A and B. Radio frequency identification (RFID) tag 810 is connected to antenna 800 with switch 820 included in the circuit, and blood glucose meter 840 has an antenna and other electronics required to read RFID tag 810. When mechanical pump 100 is in the rest state (delivery button 110 not pressed), and blood glucose meter 840 is within range of mechanical pump 110, blood glucose meter 840 detects the presence of RFID tag 810 due to wireless signal 850, as shown in Figure 8A. At this time, blood glucose meter 840 can read information stored on RFID tag 810, such as the amount of insulin delivered per press of delivery button 110, the type of insulin, and manufacturing date and identification information for mechanical pump 100.

By reading identification information for mechanical pump 100 from RFID tag 810, blood glucose meter 840 can keep track of how long the patient has used a particular pump, and alert or warn the user when it is time to change pumps to prevent mechanical pump 100 from being used beyond its intended lifetime. Now referring to Figure 8B, pressing delivery button 110 pushes activation rod 620 in the direction of the arrow, causing switch pin 830 to open switch 820, and interrupting wireless signal 850. Blood glucose meter 840 recognizes interruption in wireless signal 850 as an insulin delivery event, and records the event in its memory, along with a time stamp of the event. Blood glucose meter 840 also can display the insulin delivery event to the patient to confirm delivery and to guide the patient regarding how much insulin remains be delivered. The stored insulin delivery data also can be used to display to the patient how much insulin remains in cartridge 170. The insulin delivery data can be displayed along with blood glucose and food intake data also stored on blood glucose meter 840 to help the patient manage their blood glucose levels.

Alternatively, RFID 810, antenna 800, and switch 820 can be configured such that switch 820 is open when mechanical pump 100 is in the rest state. In this configuration, pressing delivery button 110 closes switch 820, signaling to blood glucose meter 840 that an insulin delivery event has occurred. If it is desired to increase the range with which information can be sent from mechanical pump 100 to meter 840, or increase the certainty by which the signal from mechanical pump 100 is received by meter 840, a battery can be included in the circuit with antenna 800, RFID tag 810, and switch 820, rather than relying entirely on power being supplied by meter 840 to read information from mechanical pump 100. Alternatively, a piezoelectric or other energy-generating device can be incorporated in mechanical pump 100 such that pressing delivery button 110 generates power that is used to transmit signal 850 to blood glucose meter 840. Instead of opening or closing a switch, the device could be configured such that pressing activation button 110 shields or unshields RFID tag 810, making its signal alternately detectable or undetectable by blood glucose meter 840.

It may be desirable to improve the reliability of the one-way communication between mechanical pump 100 and blood glucose meter 840. This can be accomplished by incorporating two or more RFID tags and associated antennas. For example, one RFID tag can be configured such that it can be read (i.e., detected) by meter 840 when delivery button 110 is not pressed, while a second RFID tag can be configured such that it cannot be read (i.e., detected) by meter 840 when delivery button 110 is not pressed. In such an embodiment, pressing delivery button 110 would make the first RFID tag undetectable and would make the second RFID detectable. Meter 840 would be configured to detect transitions in detectability from both tags in order to determine that a delivery event has occurred. By including additional RFID tags, a digital logic communication scheme can be easily implemented in which various tags are activated and deactivated to signal different use events.

It may be desirable to transmit more detailed information about the delivery event from mechanical pump 100 to meter 840, for example the sequential number associated with each delivery, or the delivery volume for the case where the bolus delivery volume is variable rather than fixed, for example for an insulin pen. In these cases, a more complex circuit can be included in mechanical pump 100, and two-way communication between mechanical pump 100 and meter 840 can be implemented.

In the present example, mechanical pump 100 communicates with blood glucose meter 840. However, devices other than a blood glucose meter can be used to communicate with mechanical pump 100, for example a telephone, continuous glucose monitor, remote controller, personal digital assistant, computer, or a network appliance.

In another embodiment of the present invention, mechanical pump 100 can be configured as an external device, rather than attaching it to the body via an adhesive patch. Similar to an insulin pen, the delivery mechanism can be configured to allow the user to dial in the desired dose before injecting, rather than delivering a fixed shot size with each press of the delivery button, and the mechanism can push on the plunger of the insulin cartridge, rather than using micropump 320 to suck fluid out of the reservoir. For an external device, it is important to prime the system before each use. Priming complicates the storage of bolus data, since the device must distinguish between bolus delivery and priming events. One option to address this issue is to have the blood glucose meter instruct the patient when to prime, and to record the next delivery event as a priming event. Another approach is to include a sensor on the delivery device to sense contact with the skin during a delivery event. This can be accomplished with a switch that closes when the device is brought into contact with the skin. The switch would remain open during a priming event. The status of the switch (and thus the type of event, delivery to the body or prime) would be communicated from the pump to the blood glucose meter to store with the associated delivery event in the data log.

An advantage of the present invention is the ease with which the patient can use it. To set up the pump, the patient loads a pre-filled insulin cartridge 170 into insulin cartridge compartment 150 and closes cartridge door 130. Next, the patient attaches mechanical pump 100 to adhesive patch platform 210, establishing a fluid connection between mechanical pump 100 and flexible cannula 230, and primes the device by holding down safety release button 120 and pressing delivery button 110 until a drop of insulin forms at the tip of flexible cannula 230. The patient then removes the backing from adhesive patch platform 210, secures the device to their skin, and pushes down on inserter lever 220 until it clicks in the down position. At this point the patient can deliver a fixed bolus of insulin on demand by holding down safety release button 120 and pressing delivery button 110. If desired, delivery button 110 can be configured such that upon pressing delivery button 110, the patient receives tactile and/or audible feedback to confirm that the button was pressed. Other than the simple priming step, the user does not have to perform any special steps to eliminate bubbles during setup or use of mechanical pump 100, unlike the process for a conventional insulin pump. The design of the device allows for discrete operation through clothing without the need to see the device to deliver a bolus. After depleting insulin cartridge 170, mechanical pump 100, cartridge 170, and adhesive patch platform 210 are removed and disposed of, and a new device is set up and attached to the skin. Alternatively, to reduce system cost, mechanical pump 100 can be re-used several times, reloading it with a new cartridge and attaching it to a new adhesive patch platform as necessary with each use. The device could be configured such that the patient can remove mechanical pump 100 while leaving adhesive patch platform 210 still attached to their body. This feature is useful if the patient wants to remove the pump temporarily for activities such as bathing or exercise, to change out insulin cartridges, or to check the pump if a problem is suspected. The pump can be reattached to adhesive patch platform 210 when desired by the patient.

In the case where mechanical pump 100 is removed from adhesive patch platform 210, with adhesive patch platform 210 still attached to the patient's body, it is desirable for fluid outlet from mechanical pump 100 and fluid inlet to flexible cannula 230 to be sealed when mechanical pump 100 is disconnected in order to prevent external fluid, debris, other contamination, or air from entering fluid outlet from mechanical pump 100 or fluid inlet to flexible cannula 230. In conventional infusion pumps with disconnectable infusion sets, only the infusion set portion is sealed with a septum while the needle that is connected to the pump for piercing the septum remains open and vulnerable to air and contamination. A seal can be provided on both sides by incorporating a septum on both fluid outlet from mechanical pump 100 and fluid inlet to flexible cannula 230, with a needle on one side that pierces both septums to establish a flow path when mechanical pump 100 is attached to adhesive patch platform 210.

Another advantage of the present invention is that it greatly simplifies insulin pump therapy to make it more broadly accessible while still providing beneficial safety features. Using direct micropump 320 to suck fluid from the insulin reservoir eliminates the mechanism that drives a plunger in a conventional indirect insulin pump. This greatly reduces the possibility of inadvertently driving the mechanism and over-delivering insulin. Conventional insulin pumps do not have any metering or flow-regulating device between the reservoir and the patient, making them vulnerable to failure modes such as siphoning and pressure differentials. In the present invention, micropump 320 is positioned between the insulin reservoir and the patient. Two normally closed valves 440 and 460 prevent unintentional insulin delivery. In addition, flexible diaphragm 430 is biased downwards by piston 610 in the rest position such that it presses down on and actively closes inlet valve 440, providing an extra measure of safety against over-delivery of insulin. If the drive mechanism fails or is inadvertently activated in the present invention, at most one additional bolus will be delivered. In addition, micropump 320 and fluid lines between the pump and the patient have very low compliance; thus, if an occlusion occurs, pressure in the system rises very rapidly, and it will not be possible to press delivery button 110, signaling the occlusion to the user. Safety release button 120 is an additional safety feature that prevents unintentional delivery.

Another advantage of mechanical pump 100 is that it is very small compared to existing pumps. For patients undergoing basal/bolus insulin therapy, approximately half of the insulin they inject is basal and half is bolus. If mechanical pump 100 is used to deliver only bolus insulin, or only basal insulin, the insulin reservoir can be approximately half the size of the reservoir from a conventional insulin pump used for basal/bolus therapy. If the pump is used to deliver only basal or only bolus insulin, there will be less pooling of insulin at the infusion site compared to conventional pumps which deliver both basal and bolus insulin to the same site. This may allow for the cannula to be worn longer than the typical 2-3 days before replacement. In addition, mechanical pump 100 does not have electronics, on-board power, an actuator, or a display, allowing for further size reduction. Because mechanical pump 100 is very small, it can be worn comfortably and discretely beneath clothing.

Another advantage of the present invention is that it is very low in cost compared to conventional insulin pumps, making the therapy accessible to more patients. The present invention is so low in cost that it can be disposable after each use. Thus, the user gets a new pump approximately every three days, improving reliability compared to conventional pumps which typically are expected to last for four years before replacement.

Embodiments of the present invention also employ only mechanical energy input by a user (via a user activated delivery button) to deliver a therapeutic agent (e.g., insulin) to a user. These embodiments, therefore, do not require expensive electronics or cumbersome batteries.

These and other objects and advantages of this invention will become obvious to a person of ordinary skill in this art upon reading of the detailed description of this invention including the associated drawings.

Various other modifications, adaptations, and alternative designs are of course possible in light of the above teachings. Therefore, it should be understood at this time that within the scope of the appended claims the invention might be practiced otherwise than as specifically described herein.

## Claims

1. A medical device pump comprising:
a housing with a compartment therein, the compartment configured for accommodating a cartridge containing a therapeutic agent;
a conduit configured to operatively provide a fluid flow path for therapeutic agent to exit from the cartridge;
a user activated delivery button;
a trigger mechanism, and
a mechanical pump mechanism,
wherein the trigger mechanism, user activated delivery button and mechanical pump mechanism are configured such that the trigger mechanism is activated by a user fully activating the user activated delivery button and such full activation generates mechanical power employed by, and sufficient for, the mechanical pump mechanism to pump a predetermined volume of therapeutic agent from the cartridge into the fluid flow path.

2. The medical device pump of claim 1 wherein the trigger mechanism is configured such that partial activation of the user activated delivery button by a user is insufficient to activate the trigger mechanism to generate mechanical power for the mechanical pump mechanism and, therefore, results in zero volume of therapeutic agent being pumped by the mechanical pump mechanism.

3. The medical device pump of claim 1 wherein the trigger mechanism is configured such that once a user has fully activated the user activated delivery button and the trigger mechanism has been activated, the user activated delivery button must be fully released before the trigger mechanism can be again activated.

4. The medical device pump of claim 1 further including:
a user activated safety release button configured such that pressing of the safety release button by a user enables the user activated delivery button to be fully activated by a user.

5. The medical device pump of claim 1 further including a bubble trap disposed in the fluid flow path, the bubble trap configured to prevent inadvertent bubbles in the cartridge from reaching the mechanical pump mechanism.

6. The medical device pump of claim 1 further including:
a one-way communication means, the one-way communication means configured to operatively send a communication signal from the medical device pump to an associated meter configured to detect the communication signal.

7. The medical device pump of claim 6 wherein full activation of the user activated delivery button either initiates or interrupts the sending of a communication signal from the one-way communication means to the associated meter.

8. The medical device pump of claim 6 wherein the one-way communication means is configured to be externally powered by the associated meter.

9. The medical device pump of claim 6 wherein the one-way communication means includes at least two RFID tags, and
wherein full activation of the user activated delivery button interrupts the sending of a communication signal from one of the at least two RFID tags to the associated meter, and
wherein full activation of the user activated delivery button also initiates the sending of a communication signal from another of the at least two RFID tags to the associated meter.

10. The medical device pump of claim 1 wherein the medical device is configured for multiple full activations of the user activated delivery button.

11. The medical device pump of claim 1 wherein the conduit and mechanical pump mechanism have a predetermined compliance that is sufficiently low that the user activated delivery button can not be fully activated by a user in the event of an operative occlusion in at least one of the conduit and mechanical pump mechanism.

12. A medical device kit comprising:
the medical device pump of any preceding claim wherein the compartment is configured for removably receiving the cartridge containing a therapeutic agent; and
a meter configured to detect a communication signal from the medical device pump.

13. The medical device kit of claim 12 further including an adhesive patch platform and wherein the housing is configured for removable attachment to the adhesive patch platform and wherein, optionally, the adhesive patch platform includes a flexible cannula inserter and a flexible cannula.

14. A hand-held medical fluid delivery device comprising:
a housing with a compartment therein, the compartment configured for removably receiving a cartridge containing a therapeutic agent;
a conduit configured to operatively provide a fluid flow path for therapeutic agent to exit from the cartridge;
a user activated delivery button,
a mechanical pump mechanism, and
a one-way communication means, the one-way communication means configured to operatively send a communication signal from the hand-held medical fluid delivery device to an associated meter configured to detect the communication signal,
wherein the user activated delivery button and mechanical pump mechanism are configured such that a user fully activating the user activated delivery button generates mechanical power employed by, and sufficient for, the mechanical pump mechanism to pump a predetermined volume of therapeutic agent from the cartridge into the fluid flow path.

15. A medical fluid delivery device comprising:
a housing with a compartment therein, the compartment configured for accommodating a therapeutic agent reservoir;
a conduit configured to operatively provide a fluid flow path for therapeutic agent to exit from the reservoir;
a mechanical pump mechanism; and
a bubble trap disposed in the fluid flow path, the bubble trap configured to prevent inadvertent bubbles in the cartridge from reaching the mechanical pump mechanism.
